# EUROPÄISCHE PATENTANMELDUNG

(11) **EP 4 782 457 A1**
(43) Veröffentlichungstag der Anmeldung: **29.07.2026**
(21) Anmeldenummer: 25153738.7
(22) Anmeldetag: 24.01.2025
(51) Int. Cl.: C07F 17/02, C07C 15/00, C07D 215/02

(54) **SYNTHESE UND VERWENDUNG EINES LUFTSTABILEN FERROCENYLPHOSPHASILINAN-LIGANDEN IN C X-KREUZKUPPLUNGSREAKTIONEN**

(71) Anmelder: Ludwig-Maximilians-Universität München, 80539 München (DE)
(72) Erfinder: TRAPP, Oliver, München (DE); BERTHOLD, Dino, München (DE); RAAB, Michael, München (DE)
(74) Vertreter: Weickmann & Weickmann PartmbB

(57) **Zusammenfassung**

Die vorliegende Erfindung betrifft cyclische Ferrocenylphosphasilinane und deren Verwendung als Liganden in Palladiumkomplexen bei palladium katalysierten Kreuzkupplungsreaktionen wie z.B. der Suzuki-Miyaura-Kreuzkupplung und der Buchwald-Hartwig-Kupplung.

## Beschreibung

Die vorliegende Erfindung betrifft cyclische Ferrocenylphosphasilinane und deren Verwendung als Liganden in Palladiumkomplexen bei palladium katalysierten Kreuzkupplungsreaktionen wie z.B. der Suzuki-Miyaura-Kreuzkupplung und der Buchwald-Hartwig-Kupplung.

Unter einer Kreuzkupplung wird eine Kupplungsreaktion verstanden, bei der zwei unterschiedliche Moleküle in Anwesenheit eines geeigneten Katalysators unter Ausbildung einer Kohlenstoff-Kohlenstoff- oder Kohlenstoff-Heteroatom-Bindung miteinander reagieren. Von großer praktischer Bedeutung (z.B. bei der Synthese pharmazeutischer Wirkstoffe) sind insbesondere palladiumkatalysierte Kreuzkupplungen. Beispielhaft können in diesem Zusammenhang die Stille-Kupplung, Suzuki-Kupplung (auch als Suzuki-Miyaura-Kupplung bekannt) oder Negishi-Kupplung genannt werden. Im Jahr 2010 erhielten R.F. Heck, E. Negishi und A. Suzuki den Nobelpreis für Chemie für ihre Arbeiten zu Kupplungsreaktionen.

In den folgenden Übersichtsartikeln wird die Verwendung von Kreuzkupplungsreaktionen bei der Synthese pharmazeutischer Wirkstoffe eingehender beschrieben:
- H.C. Shen, "Selected Applications of Transition Metal-Catalyzed Carbon-Carbon Cross-Coupling Reactions in the Pharmaceutical Industry", S. 25-96, in "Applications of Transition Metal Catalysis in Drug Discovery and Development: An Industrial Perspective", Ed.: M.L. Crawley and B.M. Trost, 2012, John Wiley & Sons;
- Q. Gu et al., "Palladium catalyzed C-C and C-N bond forming reactions: An update on the synthesis of pharmaceuticals from 2015-2020", Org. Chem. Front., 2021, 8, S. 384-414.

Eine weitere dem Fachmann bekannte Kupplungsreaktion ist die Buchwald-Hartwig-Kupplung, bei der ein Aryl- oder Heteroarylhalogenid oder -pseudohalogenid und ein primäres oder sekundäres Amin in Anwesenheit einer Base und eines palladiumhaltigen Katalysators unter Ausbildung einer C-N-Bindung miteinander umgesetzt werden.

In den folgenden Übersichtsartikeln wird der aktuelle Stand auf dem Gebiet der C-N-Kupplungsreaktionen zusammengefasst:
- R. Dorel et al., "The Buchwald-Hartwig Amination After 25 Years", Angew. Chem. Int. Ed., 2019, 58, S. 17118-17129;
- S.L. Buchwald et al., "Dialkylbiaryl phosphines in Pd-catalyzed amination: a user's guide", Chem. Sci., 2011, 2, S. 27-50;
- S.L. Buchwald et al., "Biaryl Phosphane Ligands in Palladium-Catalyzed Amination", Angew. Chem. Int. Ed., 2008, 47, S. 6338-6361;
- S.L. Buchwald et al., "Applications of Palladium-Catalyzed C-N Cross-Coupling Reactions", Chem. Rev., 2016 116, S. 12564-12649.

Übliche Katalysatoren auf dem Gebiet der Kupplungsreaktionen sind Palladiumkomplexe, die einen oder mehrere Phosphinliganden und optional noch weitere Liganden enthalten. Diese Pd-Komplexe mit geeigneten Phosphinliganden können vorab hergestellt und bis zu ihrer Verwendung gelagert oder alternativ *in-situ* während der zu katalysierenden Reaktion generiert werden, z. B. durch getrennte Zugabe eines Palladiumsalzes und des Phosphins zum Reaktionsmedium, so dass die Ausbildung eines phosphinhaltigen Pd-Komplexes erst im Reaktionsmedium erfolgt.

Es ist bekannt, dass nicht-cyclische Biarylphosphine als Liganden für Pd-Komplexe in palladiumkatalysierten Kupplungsreaktionen (wie z.B. der Buchwald-Hartwig-Kupplung) fungieren können, siehe z.B. S.L. Buchwald et al., 2011, supra. In diesen nicht-cyclischen Phosphinen liegt kein phosphorhaltiger Ring (d.h. kein Ring, der Phosphor als Ringatom enthält) vor.

Es sind auch cyclische Biarylphosphine (d.h. Phosphine, in denen das Phosphoratom eines der ringbildenden Atome ist) als Liganden für Pd-Komplexe in palladiumkatalysierten Kupplungsreaktionen bekannt.

S. Shekhar et al., ACS Catal., 2019, 9, S. 11691-11708, und S. Shekhar et al., ACS Catal., 2020, 10, S. 15008-15018, beschreiben Biarylphosphorinane und deren Verwendung als Pd-Komplexliganden für palladiumkatalysierte Kupplungsreaktionen.

C. Maumela et al., RSC Adv., 2021, 11, S. 26883-26891, beschreiben Biarylphobane und Biarylphosphatrioxadamantane und deren Verwendung als Pd-Komplexliganden für palladiumkatalysierte Suzuki-Kupplungen.

WO 2012/009698 A1 beschreibt ein monocyclisches, bicyclisches oder tricyclisches Biarylphosphin, wobei das heterocyclische Ringsystem neben dem Phosphoratom noch vier Kohlenstoffatome und optional mindestens ein weiteres Ringatom, das aus Kohlenstoff, Sauerstoff, Stickstoff, Phosphor und Schwefel ausgewählt ist, enthält.

Die Ausbildung einer Kohlenstoff-Heteroatom-Bindung (insbesondere einer C-N-Bindung) über eine Kupplungsreaktion, insbesondere eine Buchwald-Hartwig-Kupplung, erfordert bei der Verwendung N-heterocyclischer Arylhalogenide üblicherweise lange Reaktionszeiten. Sofern keine effizienteren Katalysatoren zur Verfügung stehen, kann beispielsweise die Reaktionstemperatur erhöht werden, um kürzere Reaktionszeiten zu realisieren. Dies führt aber üblicherweise zu unerwünschten Nebenreaktionen, die die Ausbeute des Kupplungsprodukts reduzieren.

WO 2023/088620 A1 beschreibt ein Verfahren zur Herstellung von luftstabilen Biaryl-Phosphasilinan-Liganden durch die teilweise Deprotonierung eines Phosphins und Umsetzung mit einer Dihalogenid-Verbindung. Des Weiteren wird die Anwendung von ausschließlich im Biaryl-Abschnitt Kohlenstoff-substituierten Phosphasilinan-Liganden in Pdkatalysierten Kreuzkupplungen, wie z. B. der Suzuki-Miyaura-Kreuzkupplungen und der Buchwald-Hartwig-Kupplung beschrieben.

Die Herstellung cyclischer Ferrocenylphosphasilinane kann über die Anwendung einer Giese-Reaktion erfolgen, in welcher ein Ferrocenylphosphin in einer intermolekularen und anschließenden intramolekularen Reaktion mit einem Diolefin umgesetzt wird.

Unter einer Giese-Reaktion wird die Reaktion eines Kohlenstoff- oder Heteroatom-Radikals an eine ungesättigte Kohlenstoff-Kohlenstoff-, Kohlenstoff-Heteroatom- oder Heteroatom-Heteroatom-Bindung unter Ausbildung einer neuen Kohlenstoff-Kohlenstoff-, Heteroatom-Kohlenstoff-, Kohlenstoff-Kohlenstoff- oder Heteroatom-Heteroatom-Bindung verstanden. Neben dieser Bindung wird ebenfalls eine neue Kohlenstoff-Wasserstoff- oder Heteroatom-Wasserstoff-Bindung geschlossen

In den folgenden Übersichtsartikeln wird die Giese-Reaktion eingehender beschrieben:
a) B. Giese, "Formation of CC Bonds by Addition of Free Radicals to Alkenes" Angew. Chem. Int. Ed. Engl. 1983, 22, 753-764;
b) B. Giese, "Syntheses with Radicals-C-C Bond Formation via Organotin and Organomercury Compounds [New Synthetic Methods (52)]" Angew. Chem. Int. Ed. Engl. 1985, 24, 553-565;
c) G. Bar, A. F. Parsons, "Stereoselective radical reactions" Chem. Soc. Rev. 2003, 32, 251-263;
d) G. S. C. Srikanth, S. L. Castle, "Advances in radical conjugate additions" Tetrahedron 2005, 61, 10377-10441.

Eine Aufgabe der vorliegenden Erfindung ist die Bereitstellung geeigneter, sehr elektronenreicher Phosphine, die als hochaktive Liganden in Metallkomplexen, insbesondere in Palladiumkomplexen bei palladiumkatalysierten Kreuzkupplungsreaktionen verwendbar sind. Insbesondere sollten diese Phosphine als Liganden in Palladiumkomplexen auch dann effiziente C-C-Kupplungsreaktionen, z. B. in Form einer Suzuki-Miyaura-Kupplung, oder C-N-Kupplungsreaktionen, z.B. in Form einer Buchwald-Hartwig-Kupplung, ermöglichen, wenn N-heterocyclische Arylhalogenide oder -pseudohalogenide als Reaktanten eingesetzt werden.

Ein erster Aspekt der Erfindung ist ein Phosphin der Formel **(1)** 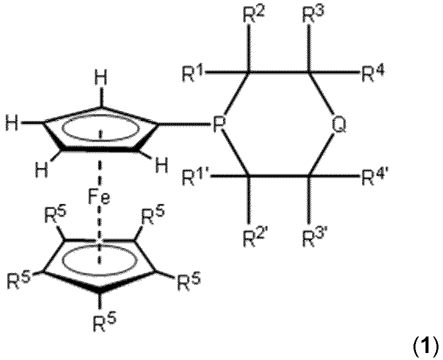 wobei
R¹, R², R³ und R⁴ unabhängig voneinander Wasserstoff, Alkyl, z.B. C₁-₄₋Alkyl, Cycloalkyl, z.B. C₅₋₇-Cycloalkyl, oder Aryl, z.B. Phenyl, sind;
R^{1'}, R^{2'}, R^{3'} und R^{4'} unabhängig voneinander Wasserstoff, Alkyl, z.B. C₁-₄₋Alkyl, Cycloalkyl, z.B. C₅₋₇-Cycloalkyl, oder Aryl, z.B. Phenyl, sind;
R⁵ jeweils unabhängig Wasserstoff, Alkyl oder Aryl ist;
Q SiR⁶R⁷, GeR⁶R⁷, SnR⁶R⁷, AsR⁶, S, SO₂ oder Se ist;
R⁶ und R⁷ unabhängig voneinander Alkyl, z.B. C₁-₄₋Alkyl, Cycloalkyl, z.B. C₅₋₇-Cycloalkyl oder Aryl, z.B. Phenyl, sind; oder R₆ und R₇ jeweils eine bivalente Alkylengruppe sind und zusammen mit einem Si-, Ge- oder Sn-Atom einen Ring, insbesondere einen 4- bis 7-gliedrigen Ring bilden.

Ein weiterer Aspekt der Erfindung ist ein Metallkomplex, insbesondere ein Palladium- oder Nickelkomplex, der ein Phosphin der Formel **(1)** als Ligand L¹ enthält.

Ein weiterer Aspekt der Erfindung ist eine Kombination, enthaltend
- eine Metallverbindung, insbesondere eine Palladium- oder Nickelverbindung und
- ein Phosphin der Formel **(1).**

Ein weiterer Aspekt der Erfindung ist die Verwendung eines Metallkomplexes, insbesondere eines Palladium- oder Nickelkomplexes, der ein Phosphin der Formel **(1)** als Ligand enthält, als Katalysator, insbesondere als Katalysator für eine Kupplungsreaktion, insbesondere für eine Kreuzkupplungsreaktion.

Noch ein weiterer Aspekt der Erfindung ist ein Verfahren zur Durchführung einer Kupplungsreaktion, wobei die Edukte der Kupplungsreaktion in Gegenwart eines erfindungsgemäßen Metallkomplexes, insbesondere eines Palladium- oder Nickelkomplexes, der ein Phosphin der Formel **(1)** enthält, oder einer erfindungsgemäßen Kombination umgesetzt werden und das Produkt der Kupplungsreaktion gegebenenfalls isoliert wird.

Der Begriff Halogen im Sinne der vorliegenden Erfindung bedeutet F, Cl, Br oder I.

Der Begriff Alkyl, insbesondere für einen der Reste R¹, R², R³, R⁴, R^{1'}, R^{2'}, R^{3'}, R^{4'} R⁵, R⁶ und R⁷ bedeutet ein geradkettiges oder verzweigtes, unsubstituiertes oder substituiertes C₁-C₁₀ Alkyl, vorzugsweise C₁-₆ Alkyl und besonders bevorzugt C₁-₄ Alkyl, wobei jedes Alkyl einen oder mehrere Substituenten unabhängig ausgewählt aus Halogen, Cycloalkyl, Aryl, O-Alkyl, O-Cycloalkyl oder O-Aryl tragen kann.

Der Begriff Cycloalkyl, insbesondere für einen der Reste R¹, R², R³, R⁴, R^{1'}, R^{2'}, R^{3'}, R^{4'} R⁵, R⁶ und R⁷ bedeutet ein C₃-₈ Cycloalkyl, vorzugsweise C₅-₇ Cycloalkyl und besonders bevorzugt C₆ Cycloalkyl, wobei jedes Cycloalkyl einen oder mehrere Substituenten unabhängig ausgewählt aus Halogen, Alkyl, O-Alkyl, oder O-Cycloalkyl, oder O-Aryl tragen kann. In bestimmten Ausführungsformen kann Cycloalkyl auch ein heterocyclisches Ringsystem mit einem oder mehreren Heteroatome im Ring, z.B. N, O oder S, darstellen. In weiteren Ausführungsformen bedeutet Cycloalkyl ein carbocyclisches Ringsystem.

Der Begriff Aryl, insbesondere für einen der Reste R¹, R², R³, R⁴, R^{1'}, R^{2'}, R^{3'}, R^{4'} R⁵, R⁶ und R⁷ bedeutet C₅-C₁₄ Aryl oder C₆-C₁₄ Aryl, vorzugsweise Phenyl oder Naphthyl und besonders bevorzugt Phenyl, wobei jedes Aryl einen oder mehrere Substituenten unabhängig ausgewählt aus Halogen, Alkyl, O-Alkyl, Cycloalkyl, O-Cycloalkyl, Aryl oder O-Aryl tragen kann. In bestimmten Ausführungsformen kann Aryl auch ein heterocyclisches Ringsystem mit einem oder mehreren Heteroatome im Ring, z.B. N, O oder S, darstellen. In weiteren Ausführungsformen bedeutet Aryl ein carbocyclisches Ringsystem.

Die cyclischen Ferrocenylphosphine der vorliegenden Erfindung enthalten in dem heterocyclischen Ring gemäß Formel **(1)** neben dem Phosphoratom noch ein weiteres Heteroatom als Ringatom, wobei dieses zusätzliche Heteroringatom Si, Ge, Sn, As oder Se ist.

R¹, R^{1'}, R², R^{2'}, R³, R^{3'}, R⁴ und R^{4'} sind vorzugsweise jeweils unabhängig ausgewählt aus H, C₁-₄₋Alkyl, C₅-₇-Cycloalkyl, oder Phenyl, das optional mit einer oder mehreren C₁₋₆-Alkylgruppen substituiert ist. Besonders bevorzugt sind R¹, R^{1'}, R², R^{2'}, R³, R^{3'}, R⁴ und R^{4'} H.

R⁵ ist vorzugsweise jeweils unabhängig ausgewählt aus H, Phenyl und Methyl.

Q ist vorzugsweise ausgewählt aus SiR⁶R⁷.

R⁶ und R⁷ sind vorzugsweise jeweils unabhängig ausgewählt aus Methyl, Phenyl, das optional mit einer oder mehreren C₁₋₆-Alkylgruppen und/oder Heteroatomgruppen, z.B. O-Alkyl, substituiert ist. Besonders bevorzugt sind R⁶ und R⁷ Phenyl, das optional mit einer O-C₁₋₆-Alkylgruppe, z.B. O-Methyl, substituiert ist.

Wie nachfolgend noch eingehender beschrieben wird, ermöglicht die Verwendung des erfindungsgemäßen Phosphins als Ligand in Metallkomplexen, z.B. in Pd-Komplexen, die Durchführung einer C-C-Kreuzkupplungsreaktion oder einer C-N-Kreuzkupplungsreaktion, beispielsweise der Buchwald-Hartwig-Kupplung, die bei relativ niedrigen Reaktionstemperaturen und relativ kurzen Reaktionszeiten zu hohen Ausbeuten führt, selbst wenn ein N-heterocyclisches Arylhalogenid oder -pseudohalogenid als Reaktant eingesetzt wird.

Der Metallkomplex, z.B. Palladium- oder Nickelkomplex, der das erfindungsgemäße Phosphin als einen Liganden enthält, kann bereits vor der zu katalysierenden Reaktion hergestellt und gegebenenfalls bis zu seiner Verwendung gelagert werden. Alternativ ist es auch möglich, dass das erfindungsgemäße Phosphin und eine als Präkursor fungierende Metallverbindung, z.B. Palladium- oder Nickelverbindung, dem Reaktionsmedium zugegeben werden, so dass die Bildung eines Metallkomplexes, z.B. Palladium- oder Nickelkomplexes, der das erfindungsgemäße Phosphin als einen Liganden enthält, *in situ* im Reaktionsmedium der Kupplungsreaktion erfolgt.

Der erfindungsgemäße Metallkomplex, z.B. Palladium- oder Nickelkomplex, der als einen Liganden L¹ das oben beschriebene erfindungsgemäße Phosphin enthält., kann zusätzlich noch einen oder mehrere, z.B. 1, 2 oder 3 Liganden L² enthalten, die jeweils kein erfindungsgemäßes Phosphin sind. Geeignete Liganden L² für Metallkomplexe, z.B. Palladium- oder Nickelkomplexe sind dem Fachmann bekannt.

Beispielsweise sind die weiteren Liganden L² des erfindungsgemäßen Metallkomplexes, z.B. Pd-Komplexes unabhängig voneinander ausgewählt aus einem Halogenid (z.B. CI⁻, Br⁻ oder I⁻); einem Aryl (z.B. Phenyl), einem Nitril (z.B. Acetonitril, Propionitril oder Benzonitril); einem Carboxylat (z.B. Acetat); einem konjugierten Dienon (z.B. ein 1,4-Dien-3-on wie Dibenzylidenaceton (dba)); einem Phosphin, das kein erfindungsgemäßes Phosphin ist (z.B. ein nicht-cyclisches Phosphin); einem Pseudohalogenid (z.B. CN⁻ oder OCN⁻) einem Amin oder Acetylacetonat.

Der Ligand L², der kein erfindungsgemäßes Phosphin ist, kann ein einzähniger oder alternativ ein mehrzähniger Ligand sein. Beispiele für solche mehrzähnigen Liganden umfassen Arylalkylamine oder Arylamine (z.B. Phenethylamin oder Naphthylamin) und Monoanionen davon.

Sofern der Ligand L² ein Phosphin ist, handelt es sich bevorzugt um ein nicht-cyclisches Phosphin, d.h. ein Phosphin, das keinen phosphorhaltigen Ring aufweist. Geeignete nicht-cyclische Phosphinliganden für Metall- bzw. Palladiumkomplexe sind dem Fachmann bekannt.

Bei dem Phosphinliganden L², der kein erfindungsgemäßes Phosphin ist, handelt es sich beispielsweise um ein Tri-C₁₋₆-Alkylphosphin, ein Tri-C₅₋₇-Cycloalkylphosphin oder ein Triarylphosphin (insbesondere ein Triphenylphosphin), wobei jede der Arylgruppen (die bevorzugt Phenylgruppen sind) optional durch eine oder mehrere C₁₋₄-Haloalkylgruppen (z.B. -CF₃), C₁₋₄-Alkylgruppen (z.B. Methyl) oder C₁₋₄-Alkoxygruppen (z.B. Methoxy) substituiert ist.

Beispielsweise weist der Phosphinligand L² eine der folgenden Formeln **(2), (3)** oder **(4)** auf:

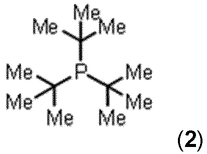

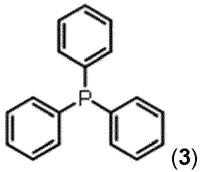

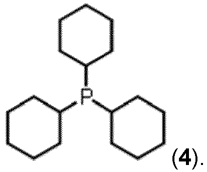

Alternativ kann es sich bei dem Phosphinliganden L² um ein Phosphin der folgenden Formel **(5)** handeln:

P(R¹)(R²)(R³) **(5)**

wobei R¹ und R² unabhängig voneinander ausgewählt sind aus Alkyl, insbesondere C₁₋₆-Alkyl und Cycloalkyl, insbesondere C₅₋₇-Cycloalkyl (z.B. Cyclohexyl), und R³ Biphenyl ist, das optional durch eine oder mehrere C₁₋₆-Alkylgruppen, C₁₋₆-Alkoxygruppen, Phenylgruppen und/oder Pyridylgruppen substituiert ist.

Beispielsweise weist der Phosphinligand L² eine der folgenden Formeln **(6), (7), (8)** oder **(9)** auf:

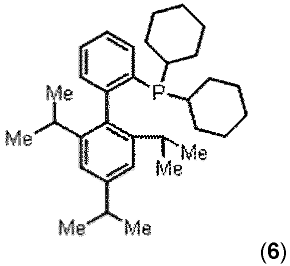

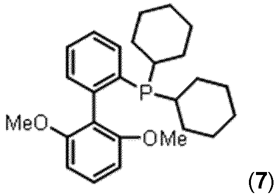

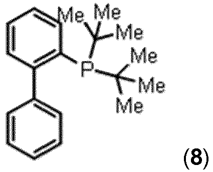

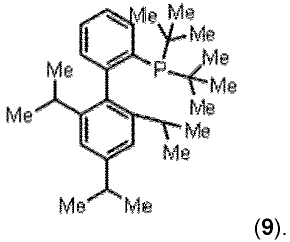

Ein erfindungsgemäßer Palladiumkomplex weist beispielsweise die folgende Formel **(10)** auf: 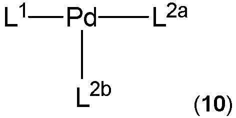 wobei
L¹ ein erfindungsgemäßes Phosphin ist, und
L^{2a} und L^{2b} jeweils ein Ligand ist, der kein erfindungsgemäßes Phosphin ist.

Hinsichtlich geeigneter Liganden L^{2a} und L^{2b} kann auf die obigen Ausführungen zu dem Liganden L² verwiesen werden. Beispielsweise sind die Liganden L^{2a} und L^{2b} des erfindungsgemäßen Pd-Komplexes unabhängig voneinander ausgewählt aus einem Halogenid (z.B. CI⁻, Br⁻ oder I⁻); einem Aryl; einem Nitril (z.B. Acetonitril, Propionitril oder Benzonitril); einem Carboxylat (z.B. Acetat); einem konjugierten Dienon (z.B. ein 1,4-Dien-3-on wie Dibenzylidenaceton (dba)); einem Phosphin, das kein erfindungsgemäßes Phosphin ist (z.B. ein nicht-cyclisches Phosphin); einem Pseudohalogenid (z.B. CN⁻ oder OCN⁻) einem Amin oder Acetylacetonat.

Ein beispielhafter Palladiumkomplex der vorliegenden Erfindung weist die folgende Formel **(11)** auf: 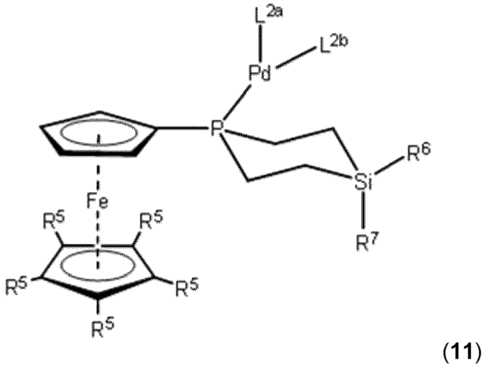 wobei R⁵, R⁶ und R⁷ sowie die Liganden L^{2a} und L^{2b} die oben angegebenen Bedeutungen aufweisen.

Beispielsweise ist der Ligand L^{2a} Dibenzylidenaceton (dba) oder Acetonitril und der Ligand L^{2b} wird aus einem der oben für L² angegebenen Liganden ausgewählt.

Ein weiterer beispielhafter Palladiumkomplex der vorliegenden Erfindung weist die folgende Formel **(12)** auf: 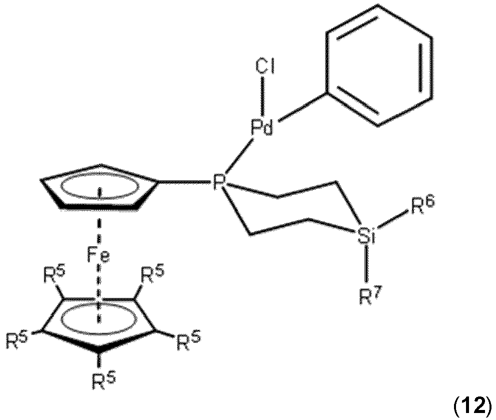 wobei R⁵, R⁶ und R⁷ die oben angegebenen Bedeutungen aufweisen.

Ein weiterer beispielhafter Palladiumkomplex der vorliegenden Erfindung weist die folgende Formel **(13)** auf: 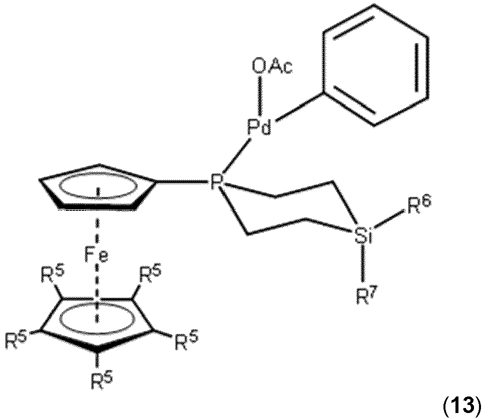 wobei R⁵, R⁶ und R⁷ die oben angegebenen Bedeutungen aufweisen.

Wie oben bereits erwähnt, können das erfindungsgemäße Phosphin und eine als Präkursor fungierende Metall- bzw. Palladiumverbindung, die das erfindungsgemäße Phosphin noch nicht enthält, dem Reaktionsmedium zugegeben werden, so dass die Bildung eines Metall- bzw. Palladiumkomplexes, der das erfindungsgemäße Phosphin als einen Liganden enthält, *in-situ* im Reaktionsmedium der Kupplungsreaktion erfolgt.

Die vorliegende Erfindung betrifft daher auch eine Kombination, enthaltend
- eine Metall- bzw. Palladium- oder Nickelverbindung und
- das oben beschriebene erfindungsgemäße Phosphin.

Die Metall- bzw. Palladium- oder Nickelverbindung der erfindungsgemäßen Kombination enthält üblicherweise kein erfindungsgemäßes Phosphin.

Die Kombination kann in einem Behälter vorliegen, der sowohl die Metall- bzw. Palladium- oder Nickelverbindung als auch das erfindungsgemäße Phosphin enthält. Optional kann die Kombination in Form eines Kits, der die Metall- bzw. Palladium- oder Nickelverbindung und das erfindungsgemäße Phosphin in getrennten Behältern enthält, vorliegen.

Die Metall- bzw. Palladiumverbindung ist beispielsweise ein Metall- bzw. Palladiumsalz oder ein Metall- bzw. Palladiumkomplex, dessen Liganden kein erfindungsgemäßes Phosphin sind.

Das Metall- bzw. Palladiumsalz ist beispielsweise ein Palladiumacetat, ein Palladiumhalogenid (z.B. ein Palladiumchlorid, Palladiumbromid oder Palladiumiodid) oder ein Palladiumpseudohalogenid oder ein Gemisch aus mindestens zwei dieser Salze.

Handelt es sich bei der Metall- bzw. Palladiumverbindung um einen Metall- bzw. Palladiumkomplex, so sind dessen Liganden unabhängig voneinander beispielsweise ausgewählt aus einem Halogenid (z.B. CI⁻, Br⁻ oder I⁻); einem Phosphin, das kein erfindungsgemäßes Phosphin ist (z.B. ein nicht-cyclisches Phosphin); einem konjugierten Dienon, z.B. einem 1,4-Dien-3-on wie Dibenzylidenaceton (dba); einem Nitril, z.B. Acetonitril, Propionitril oder Benzonitril; einem Acetylacetonat; einem Carboxylat, z.B. Acetat; einem Pseudohalogenid, z.B. CN⁻ oder OCN⁻, einem Amin oder einem Aryl.

Hinsichtlich des Phosphinliganden, der kein erfindungsgemäßes Phosphin ist, kann auf die obigen Ausführungen zu Ligand L² verwiesen werden. Bevorzugt ist der Phosphinligand, der kein erfindungsgemäßes Phosphin ist, ein nicht-cyclisches Phosphin, d.h. ein Phosphin, das keinen phosphorhaltigen Ring aufweist. Geeignete nicht-cyclische Phosphinliganden für Palladiumkomplexe sind dem Fachmann bekannt. Bei dem Phosphinliganden, der kein erfindungsgemäßes Phosphin ist, handelt es sich beispielsweise um ein Tri-C₁₋₆-Alkylphosphin, ein Tri-C₅₋₇-Cycloalkylphosphin oder ein Triarylphosphin, insbesondere ein Triphenylphosphin, wobei jede der Arylgruppen, die bevorzugt Phenylgruppen sind, optional durch eine oder mehrere C₁₋₄-Haloalkylgruppen (z.B. -CF₃), C₁₋₄-Alkylgruppen (z.B. Methyl) und/oder C₁₋₄ -Alkoxygruppen (z.B. Methoxy) substituiert ist. Alternativ kann es sich bei dem Phosphinliganden, der kein erfindungsgemäßes Phosphin ist, beispielsweise um ein Phosphin der oben beschriebenen Formel **(8)** handeln.

Als beispielhafte Palladiumverbindungen der erfindungsgemäßen Kombination können Folgende genannt werden: ein Palladiumdibenzyliden-Komplex (z.B. Pd₂(dba)₃ oder Pd(dba)₂); PdCl₂(PR₃)₂, wobei R ein Phenyl (das optional mit einer oder mehreren C₁₋₆-Alkylgruppen substituiert ist), ein C₅₋₇-Cycloalkyl oder ein C₁₋₆-Alkyl ist; ein Palladiumacetat (z.B. Pd₂(OAc)₃); ein Palladiumacetylacetonat; ein PdX₂, wobei X ein Halogenid oder Pseudohalogenid ist; ein Pd(RCN)₂Cl₂, wobei R ein Phenyl oder Methyl ist.

Die vorliegende Erfindung betrifft außerdem die Verwendung des oben beschriebenen erfindungsgemäßen Metallkomplexes, z.B. Palladium- oder Nickelkomplexes, oder der oben beschriebenen erfindungsgemäßen Kombination als Katalysator, insbesondere als Katalysator in einer Kreuzkupplungsreaktion.

Die Kreuzkupplungsreaktion ist beispielsweise eine C-C- oder C-N-Kreuzkupplungsreaktion. Die C-C-Kreuzkupplungsreaktion ist beispielsweise eine Suzuki-Miyaura-Kupplung. Wie dem Fachmann bekannt ist, handelt es sich bei der Suzuki-Miyaura-Kupplung um eine Kupplungsreaktion, bei der eine Organobor-Verbindung und beispielsweise ein Aryl- oder Heteroarylhalogenid, -pseudohalogenid oder -sulfonat in Anwesenheit eines Katalysators, z.B. eines palladiumhaltigen Katalysators, unter Ausbildung einer C-C-Bindung miteinander umgesetzt werden.

Eine bevorzugte C-N-Kreuzkupplungsreaktion ist die Buchwald-Hartwig-Kupplung. Wie dem Fachmann bekannt ist, handelt es sich bei der Buchwald-Hartwig-Kupplung um eine Kupplungsreaktion, bei der ein Aryl- oder Heteroarylhalogenid, -pseudohalogenid oder - sulfonat und ein primäres oder sekundäres Amin in Anwesenheit eines Katalysators, z.B. eines palladiumhaltigen Katalysators, (und bevorzugt einer Base) unter Ausbildung einer C-N-Bindung miteinander umgesetzt werden

Die vorliegende Erfindung betrifft außerdem ein Verfahren zur Herstellung eines Aryl- oder Heteroarylamins, wobei eine Verbindung der Formel **(14)**

Ar-X **(14)**

wobei
Ar ein Aryl oder Heteroaryl ist,
X ein Halogenatom, eine Sulfonatgruppe (z.B. Trifluormethansulfonat-O-Tf) oder eine Pseudohalogengruppe (z.B. -CN, -OCN oder -NCO) ist, mit einem primären oder sekundären Amin in Anwesenheit des oben beschriebenen erfindungsgemäßen Metallokomplexes, z.B. Palladium- oder Nickelkomplexes, oder der oben beschriebenen erfindungsgemäßen Kombination umgesetzt wird.

Geeignete Reaktionsbedingungen für die Buchwald-Hartwig-Kupplung sind dem Fachmann bekannt. Bevorzugt erfolgt die Umsetzung in Anwesenheit einer Base.

Im Folgenden soll die Erfindung durch die nachfolgenden Beispiele näher erläutert werden.

### Beispiele

### Herstellung eines erfindungsgemäßen cyclischen Ferrocenylphosphins

Ein erfindungsgemäßes Ferrocenyl-Phosphasilinan der folgenden Formel **(15)** 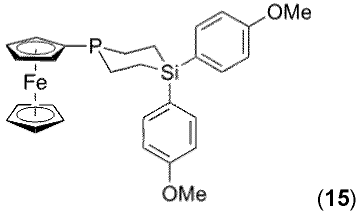 wurde nach dem folgenden Reaktionsschema hergestellt:

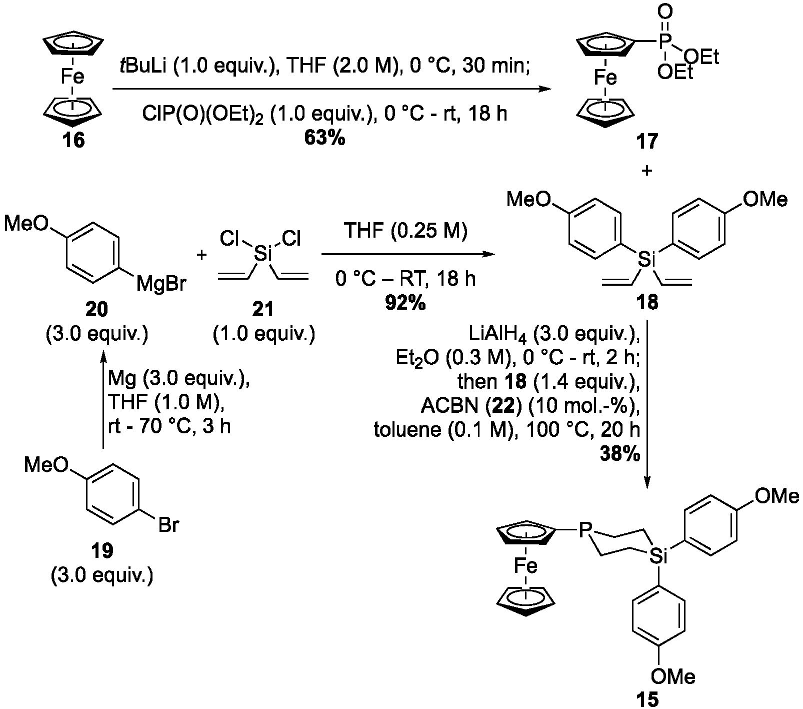

Chemischer Name des cyclischen Biarylphosphins der Formel **(15):**
4,4-Bis(4-methoxyphenyl)-1-ferrocenyl-1,4-phosphasilinan

### 1.1

### Diethylferrocenylphosphonat (17)

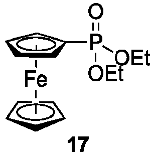

Ferrocen (4.65 g, 25.0 mmol, 1.00 equiv.) wurde bei 0°C in THF (12.5 mL, 2.0 _{M}) vorgelegt und tBuLi(1.70 M in *n*Pentan, 14.7 mL, 25.0 mmol, 1.00 equiv.) über 5 min zugegeben. Diese Lösung wurde für 20 min gerührt, dann wurde Diethylchlorphosphat (3.64 mL, 25.0 mmol, 1.00 equiv.) zugegeben und für 30 min gerührt. Die Reaktionslösung wurde auf Raumtemperatur (RT) erwärmt und für 18 h gerührt. Dann wurde MeOH (5 mL) zugegeben, das Lösungsmittel bei vermindertem Druck entfernt. Das Rohprodukt wurde anschließend säulenchromatografisch (SiOz, Et₂O - EtOAc - EtOAc:EtOH = 20:1 - 10:1) aufgereinigt. Das Produkt **(17)** wurde als rotbraunes Öl (5.13 g, 15.9 mmol, 63%) erhalten.

**Rf** (SiO₂, EtOAc: EtOH = 10:1) = 0.65.

**¹H NMR** (401 MHz, CDCl₃): δ = 4.49-4 .52 (m, 2H), 4.38-4.41 (m, 2H), 4.31 (s, 5H), 4.08-4.15 (m, 4H,), 1.34 (t, ³J_{H,H} = 6.4 Hz, 6H) ppm.

**³¹P NMR** (162 MHz, CDCl₃): δ = 25.8 (s, 1P) ppm.

### 12

### Divinylbis(para-methoxyphenyl)silan (18)

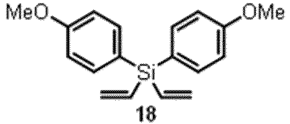

Mg-Späne (3.65 g, 150 mmol, 6.00 equiv.) wurden in einem Kolben vorgelegt und ausgeheizt. Anschließend wurde mit THF (5 mL) übergeschichtet und *para*-Bromanisol (9.41 mL, 75.0 mmol, 3.00 equiv.) zusammen mit THF (70 mL, 2.0 _{M}) portionsweise zugegeben. Diese Lösung wurde 2 h bei 80°C refluxiert. Divinyldichlorsilan (3.55 mL, 25.0 mmol, 1.00 equiv.) wurde in THF (25 mL, 1.0 _{M}) bei 0°C gelöst und die Grignard-Lösung zugegeben. Die resultierende Lösung wurde 1 h bei 0°C und 16 h bei 23°C gerührt. Die Reaktionsmischung wurde mit H₂O (100 mL) gequencht und die wässrige Phase mit Et₂O (3 x 50 mL) extrahiert. Die vereinigten organischen Phasen wurden über MgSO4 getrocknet und das Lösungsmittel bei vermindertem Druck entfernt. säulenchromatografisch (SiOz, *n*Pentan:Et₂O 20:1 - 10:1) aufgereinigt. Das Produkt **(18)** wurde als gelbliches Öl (6.45 g, 23.1 mmol, **92%)** erhalten.

**Rf** (SiO₂, *n*Pentan:Et₂O = 20:1) = 0.25.

**¹H NMR** (401 MHz, CDCl₃): δ = 7.45 (d, ²*J*_{H,H} = 8.8 Hz, 4H), 6.92 (d, ²*J*_{H,H} = 8.8 Hz, 4H), 6.20-6.52 (m, 4H), 5.74-5.81 (m, 2H), 3.82 (s, 6H, OCH₃) ppm.

### 1.3

### 4,4-Bis(4-methoxyphenyl)-1-ferrocenyl-1,4-phosphasilinan (15)

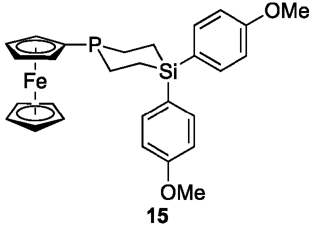

LiAlH₄ (2.28 g, 60.0 mmol, 3.0 equiv.) wurde bei 0°C in Et₂O (46.7 mL) vorgelegt und langsam mit einer Lösung von **17** (6.44 g, 20.0 mmol, 1.00 equiv.) in Et₂O (20 mL) versetzt. Diese Lösung wurde für 1 h bei 0°C und anschließend für 18 h bei RT gerührt. Dann wurde die Lösung auf 0°C gekühlt, mit Na₂So₄·10 H₂O (80 g) versetzt, 1 h bei 0 °C und 3 h bei RT gerührt. Das Gemisch wurde anschließend über eine Fritte filtriert, der Rückstand wurde mit Et₂O (200 mL) gewaschen und das Filtrat anschließend bei vermindertem Druck eingeengt. Der Rückstand wurde in Toluol (200 mL) gelöst und mit 1,1'-Azobis(cyclohexancarbonitril) **(22)** (489 mg, 2.00 mmol, 10.0 mol.-%) und **18** (8.29 g, 28.0 mmol, 1.4 equiv.) versetzt. Diese Reaktionsmischung wurde für 20 h bei 100°C erhitzt. Das Lösungsmittel wurde bei vermind. Druck entfernt und der Rückstand säulenchromatografisch (SiO₂, *n*Pentan:EtOAc = 10:1) aufgereinigt. Das Produkt **(15)** wurde als orangener Feststoff (3.90 g, 7.58 mmol, 38%) erhalten.

Rf (SiO₂, *n*Pentan:EtOAc = 10:1) = 0.50.

**¹H NMR** (801 MHz, C₆D₆): δ = 7.67 (d, ²*J*_{H,H} = 8.8 Hz, 2H), 7.56 (d, ²*J*_{H,H} = 8.8 Hz, 2H), 7.02 (d, ²*J*_{H,H} = 8.0 Hz, 2H), 6.93 (d, ²*J*_{H,H} = 8.0 Hz, 2H), 4.20-4.22 (m, 2H), 4.13-4.15 (m, 2H), 4.12 (s, 5H), 3.41 (s, 3H), 3.38 (s, 3H), 2.30-2.34 (m, 2H), 2.10-2.16 (m, 2H), 1.76-1.84 (m, 2H), 1.62-1.69 (m, 2H) ppm.

**¹³C NMR** (201 MHz, C₆D₆): δ = 161.6 (s, 1C), 161.5 (s, 1C), 136.8 (s, 2C), 136.2 (s, 2C), 128.5 (s, 2C) 114.7 (s, 2C), 114.3 (s, 2C), 80.1 (d, ¹*J*_{C,P} = 14 Hz, 1C), 71.3 (d, ²*J*_{C-H} = 13 Hz, 2C), 70.2 (d, ³*J*_{C-P} = 3.6 Hz, 2C), 69.2 (s, 5C), 54.7 (s, 1C), 54.7 (1s, 1C), 24.7 (d, ¹J_{C-P} = 11 Hz, 2C), 10.0 (s, 1C), 9.95 (s, 1C) ppm.

**³¹P NMR** (256 MHz, C₆D₆): δ = -34.0 (s, 1P).

### Verwendung einer Zusammensetzung, die ein erfindungsgemäßes Ferrocenylphosphasilinan und eine Palladiumverbindung enthält, als Katalysator in einer Suzuki-Miyaura-Kreuzkupplung

In den nachfolgend beschriebenen erfindungsgemäßen Beispielen 1-12 wurde das Ferrocenylphosphasilinan der Formel **(15)** verwendet, d.h.:

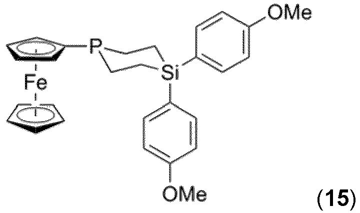

In dem Vergleichsbeispiel 1 wurde ein nicht-cyclisches Ferrocenylphosphin der folgenden Formel **(23)** verwendet:

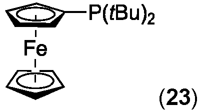

Unter Verwendung eines erfindungsgemäßen Ferrocenylphosphins der Formel (15) wurde in den erfindungsgemäßen Beispielen 1-12 eine Suzuki-Miyaura-Kupplung mit den im nachfolgenden Reaktionsschema und in der Tabelle 1 angegebenen Reaktanten und Reaktionsbedingungen durchgeführt. Die Ausbeuten sind in der Tabelle 1 angegeben.

In Beispiel 1 wurden 4-Chlorchinolin und Phenylboronsäure als Reaktanten für die Suzuki-Miyaura Kreuzkupplung verwendet.

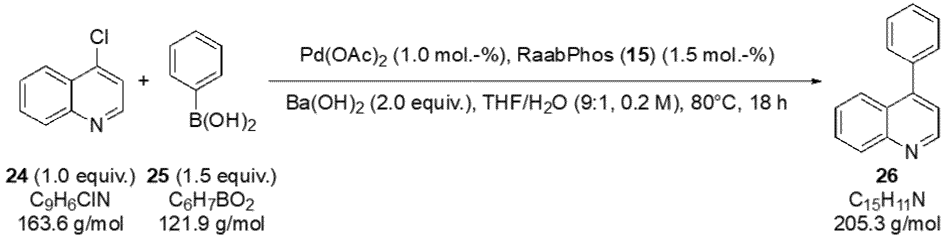

### Beispiele 1-12 und Vergleichsbeispiel 1:

Pd(OAc)₂ (1.12 mg, 5.00 µmol, 1.00 mol.-%) und das Ferrocenylphosphin der Formel **15** (3.86 mg, 7.50 µmol, 1.50 mol.-%) wurden in einem entgastem Gemisch von THF/H₂O (10:1, 2.5 mL, 0.2 _{M}) bei RT vorgelegt und 10 min gerührt. Anschließend wurde 4-Chlorchinolin **(24)** (81.8 mg, 0.500 mmol, 1.00 equiv.), Phenylboronsäure **(25)** (91.4 mg, 0.750 mmol, 1.50 equiv.) und Ba(OH)₂ (171 mg, 1.00 mmol, 2.00 equiv.) hinzugegeben. Die Reaktionsmischung wurde für 6 h bei 80 °C erhitzt. Das Lösungsmittel wurde bei vermindertem Druck entfernt. Das Produkt wurde durch Säulenchromatografie (C₁₈-SiO₂, MeCN/H₂O = 5:95 - 100:0) aufgereinigt. 4-(Phenyl-1-yl)chinolin **(26)** (99.0 mg 0.482 mmol, **96%)** wurde als farbloser Feststoff isoliert werden.

In den erfindungsgemäßen Beispielen 1-12 wurden die Reaktanten variiert (siehe nachfolgende Tabelle 1), die Synthesebedingungen waren jedoch identisch mit den in Beispiel 1 verwendeten Synthesebedingungen.

In Vergleichsbeispiel 1 waren die Reaktanten und die Synthesebedingungen identisch mit den in Beispiel 1 verwendeten Reaktanten und Synthesebedingungen. Allerdings wurde anstelle des erfindungsgemäßen Phosphins der Formel (15) das Phosphin der Formel (23) verwendet.

Die Ergebnisse der erfindungsgemäßen Beispiele 1-12 sind in der nachfolgenden Tabelle 1 zusammengefasst.

**Tabelle 1: Als Reaktanten verwendete Boronsäuren, bei der Suzuki-Miyaura-Kreuzkupplung erhaltene Reaktionsprodukte und Produktausbeuten in den erfindungsgemäßen Beispielen 1-12.**

| Beispiel | Heteroaryl | Boronsäure | Reaktionsprodukt | Ausbeute [%] |
|---|---|---|---|---|
| 1 | 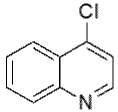 | 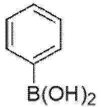 | 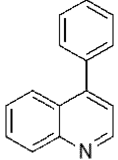 | 96% |
| 2 | 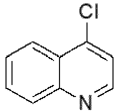 | 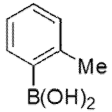 | 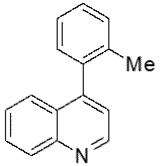 | 90% |
| 3 | 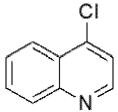 | 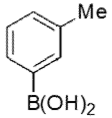 | 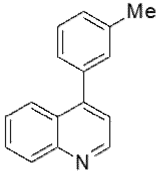 | 98% |
| 4 | 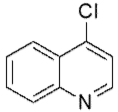 | 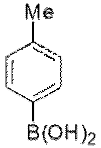 | 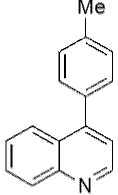 | 98% |
| 5 | 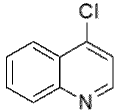 | 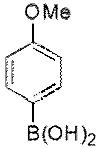 | 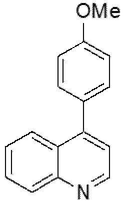 | 90% |
| 6 | 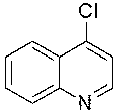 | 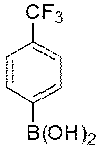 | 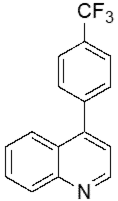 | 93% |
| 7 | 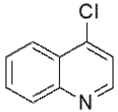 | 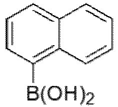 | 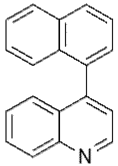 | 95% |
| 8 | 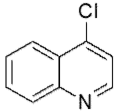 | 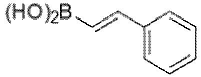 | 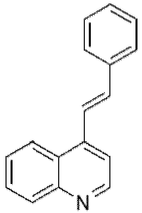 | 91% |
| 9 | 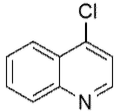 | 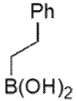 | 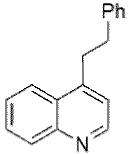 | 76% |
| 10 | 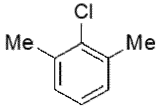 | 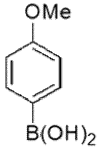 | 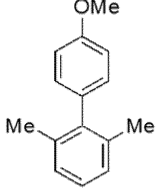 | 95% |
| 11 | 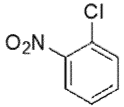 | 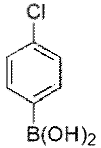 | 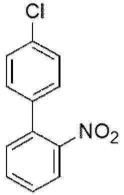 | 87% |
| 12 | 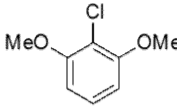 | 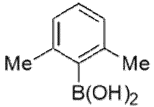 | 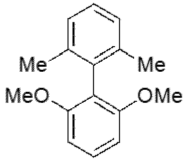 | 91% |

Obwohl in vielen Beispielen einer der Reaktanten ein N-heterocyclisches Arylchlorid war und eine recht kurze Reaktionsdauer bei relativ milder Reaktionstemperatur gewählt wurde, führte die Verwendung des erfindungsgemäßen cyclischen Biarylphosphins als Ligand eines Palladiumkomplexes bei einer palladiumkatalysierten Suzuki-Miyaura-Kreuzkupplung zu hohen Produktausbeuten.

Das Ergebnis des Vergleichbeispiels 1 ist in der nachfolgenden Tabelle 2 angegeben.

**Tabelle 2: Als Reaktanten verwendetes Phenylboron und Produktausbeute bei der Suzuki-Miyaura-Kreuzkupplung im Vergleichbeispiel 1.**

| | Heteroaryl | Boronsäure | Reaktionsprodukt | Ausbeute |
|---|---|---|---|---|
| Vergleich beispiel 1 | 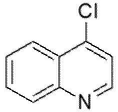 | 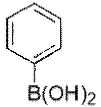 | 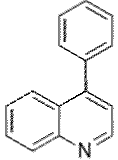 | 38% |

In Vergleichsbeispiel 1 war die Ausbeute des Produkts mit 38% ganz erheblich geringer als im erfindungsgemäßen Beispiel 1 mit 96%.

### Verwendung von Zusammensetzungen, die ein erfindungsgemäßes Ferrocenylphosphasilinan und eine Palladiumverbindung enthalten, als Katalysator in einer Buchwald-Hartwig-Kupplung

In den nachfolgend beschriebenen erfindungsgemäßen Beispielen 13-20 wurde das Ferrocenylphosphasilinan der Formel (15) verwendet, d.h.:

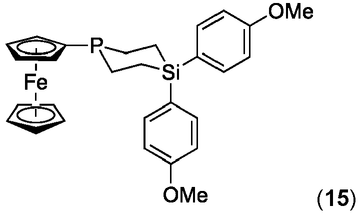

In dem Vergleichsbeispiel 2 wurde ein nicht-cyclisches Ferrocenylphosphin der folgenden Formel **(23)** verwendet:

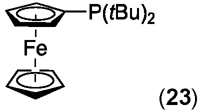

Als Palladiumverbindung wurde in allen Beispielen (d.h. den erfindungsgemäßen Beispielen 13-20 und dem Vergleichsbeispiel 2 ein Palladiumdibenzylidenkomplex (Pd₂(dba)₃) verwendet. Diese Palladiumverbindung und das Phosphin der Formel **(15)** oder **(23)** wurden dem Reaktionsmedium zugegeben, so dass sich *in-situ* ein Palladiumkomplex, der das Phosphin als einen seiner Liganden enthält, bilden konnte.

In Beispiel 13 wurden 4-Chlorchinolin und Pyrrolidin als Reaktanten für die Buchwald-Hartwig Kupplung verwendet.

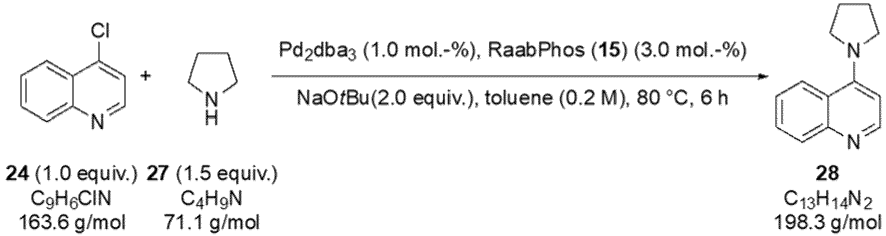

### Beispiele 13-20 und Vergleichsbeispiel 2:

In Beispiel 13 wurden Pd₂dba₃ (4.56 mg, 5.00 µmol, 1.00 mol.-%) und das Ferrocenylphosphin der Formel **15** (7.72 mg, 150 µmol, 3.00 mol.-%) in entgastem und trockenem Toluol (2.5 mL, 0.2 _{M}) bei RT vorgelegt und 10 min gerührt. Anschließend wurde 4-Chlorchinolin **(24)** (81.8 mg, 0.500 mmol, 1.00 equiv.), NaO*t*Bu (96.1 mg, 1.00 mmol, 2.00 equiv.) und Pyrrolidin **(27)** (62.0 µL, 53.3 mg, 0.750 mmol, 1.50 equiv.) hinzugegeben. Die Reaktionsmischung wurde für 6 h bei 80 °C erhitzt. Das Lösungsmittel wurde bei vermindertem Druck entfernt. Das Produkt wurde durch Säulenchromatografie (C18-SiO₂, MeCN/H₂O = 5:95 - 100:0) aufgereinigt. 4-(Pyrrolidin-1-yl)chinolin **(28)** (98.8 mg 0.498 mmol, **99%)** wurde als farbloser Feststoff isoliert.

In den erfindungsgemäßen Beispielen 14-20 wurden die Reaktanten variiert (siehe nachfolgende Tabelle 2), die Synthesebedingungen waren jedoch identisch mit den in Beispiel 13 verwendeten Synthesebedingungen.

In Vergleichsbeispiel 2 waren die Reaktanten und die Synthesebedingungen identisch mit den in Beispiel 13 verwendeten Reaktanten und Synthesebedingungen. Allerdings wurde anstelle des erfindungsgemäßen Phosphins der Formel **(15)** das Phosphin der Formel **(23)** verwendet.

Die Ergebnisse der erfindungsgemäßen Beispiele 13-20 sind in der nachfolgenden Tabelle 3 zusammengefasst.

**Tabelle 3: Als Reaktanten verwendete Amine, bei der Buchwald-Hartwig-Kupplung erhaltene Reaktionsprodukte und Produktausbeuten in den erfindungsgemäßen Beispielen 13-20.**

| | | | | |
|---|---|---|---|---|
| 13 | 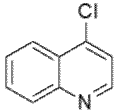 | 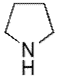 | 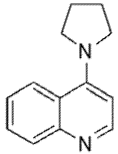 | 99% |
| 14 | 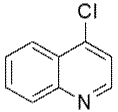 | 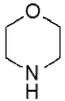 | 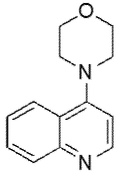 | 96% |
| 15 | 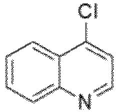 | 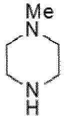 | 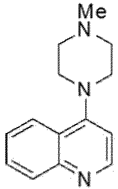 | 79% |
| 16 | 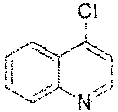 | PhNHMe | 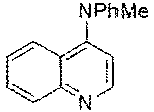 | 81% |
| 17 | 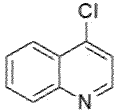 | NH₃ | 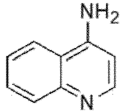 | 81% |
| 18 | 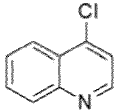 | 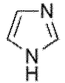 | 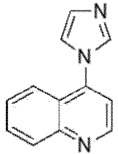 | 95% |
| 19 | 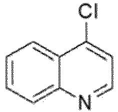 | 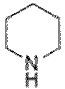 | 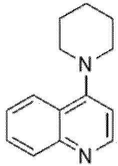 | 97% |
| 20 | 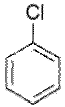 | PhNHMe | 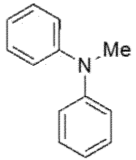 | 91% |

Obwohl in allen Beispielen einer der Reaktanten ein N-heterocyclisches Arylchlorid war und eine recht kurze Reaktionsdauer bei relativ milder Reaktionstemperatur gewählt wurde, führte die Verwendung des erfindungsgemäßen cyclischen Biarylphosphins als Ligand eines Palladiumkomplexes bei einer palladiumkatalysierten Buchwald-Hartwig-Kupplung zu hohen Produktausbeuten.

Das Ergebnis des Vergleichsbeispiels 2 ist in der nachfolgenden Tabelle 4 angegeben.

**Tabelle 4: Als Reaktanten verwendete Amine und Produktausbeute bei der Buchwald-Hartwig-Kupplung im Vergleichsbeispiel 2.**

| | Heteroaryl | Amin | Reaktionsprodukt | Ausbeute |
|---|---|---|---|---|
| Vergleichsbeispiel 2 | 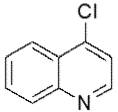 | 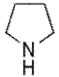 | 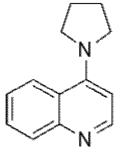 | 60% |

In Vergleichsbeispiel 2 war die Ausbeute des Produkts mit 60% ganz erheblich geringer als im erfindungsgemäßen Beispiel 13 mit 99%.

## Patentansprüche

1. Ferrocenylphosphasilinan der Formel **(1)** 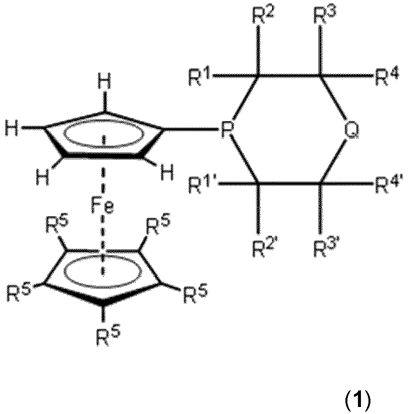 wobei
R¹, R², R³ und R⁴ unabhängig voneinander Wasserstoff, Alkyl, Cycloalkyl oder Aryl sind;
R^{1'}, R^{2'}, R^{3'} und R^{4'} unabhängig voneinander Wasserstoff, Alkyl, Cycloalkyl oder Aryl sind;
R⁵ jeweils unabhängig Wasserstoff, Alkyl oder Aryl ist;
Q SiR⁶R⁷, GeR⁶R⁷, SnR⁶R⁷, AsR⁶, S, SO₂ oder Se ist;
R⁶ und R⁷ unabhängig voneinander Alkyl, Cycloalkyl oder Aryl sind; oder R₆ und R₇ jeweils eine bivalente Alkylengruppe sind und zusammen mit einem Si-, Ge- oder Sn-Atom einen Ring bilden.

2. Metallkomplex, der als einen Liganden L¹ das Phosphin gemäß dem Anspruch 1 enthält.

3. Metallkomplex nach Anspruch 2, der ein Palladium- oder Nickelkomplex ist.

4. Metallkomplex nach Anspruch 2 oder 3, der zusätzlich mindestens einen Liganden L² enthält, der jeweils kein Phosphin gemäß dem Anspruch 1 ist.

5. Metallkomplex nach Anspruch 4, wobei der mindestens eine Ligand L² unabhängig voneinander ausgewählt ist aus einem Halogenid; einem Aryl; einem Nitril; einem Carboxylat; einem konjugierten Dienon; einem Phosphin, das kein Phosphin nach Anspruch 1 ist; einem Amin oder Acetylacetonat.

6. Kombination, enthaltend
- eine Metallverbindung, insbesondere eine Palladium- oder Nickelverbindung, und
- ein Phosphin der Formel **(1).**

7. Kombination nach Anspruch 6, wobei die Bestandteile der Kombination gemeinsam in einem Behälter oder separat in mehreren Behältern vorliegen.

8. Kombination nach Anspruch 6 oder 7, wobei die Metallverbindung, insbesondere die Palladium- oder Nickelverbindung, ausgewählt ist aus einem Metallsalz, insbesondere einem Palladium- Nickelsalz, einem Metallkomplex, insbesondere einem Palladium- oder Nickelkomplex, der kein Phosphin gemäß Anspruch 1 als Liganden enthält, oder einer Kombination von mehreren Salzen und/oder Komplexen.

9. Kombination nach Anspruch 8, wobei das Metallsalz ein Palladiumsalz ausgewählt aus einem Palladiumacetat, einem Palladiumhalogenid, einem Palladiumpseudohalogenid oder einem Gemisch aus mindestens zwei dieser Salze ist.

10. Kombination nach Anspruch 8, wobei der Metallkomplex, insbesondere der Palladium- oder Nickelkomplex, einen oder mehrere Liganden, unabhängig voneinander ausgewählt aus einem Halogenid; einem Phosphin, das kein Phosphin gemäß Anspruch 1 ist; einem konjugierten Dienon, vorzugsweise einem 1,4-Dien-3-on wie Dibenzylidenaceton; einem Nitril; einem Acetylacetonat; einem Carboxylat; einem Pseudohalogenid; einem Amin; einem Aryl enthält.

11. Verwendung eines Metallkomplexes, insbesondere eines Palladium- oder Nickelkomplexes gemäß einem der Ansprüche 2 - 5 oder einer Kombination gemäß einem der Ansprüche 6 bis 10 als Katalysator, z.B. als Katalysator in einer Kupplungsreaktion, insbesondere in einer Kreuzkupplungsreaktion.

12. Verwendung nach Anspruch 11, wobei die Kreuzkupplungsreaktion eine C-C-Kreuzkupplungsreaktion, insbesondere eine Suzuki-Miyaura-Kreuzkupplung, oder eine C-N-Kreuzkupplungsreaktion, insbesondere eine Buchwald-Hartwig-Kupplung, ist.

13. Verfahren zur Durchführung einer Kupplungsreaktion, umfassend:
- Umsetzen der Edukte der Kupplungsreaktion in Anwesenheit eines Metallkomplexes, insbesondere eines Palladium- oder eines Nickelkomplexes, gemäß einem der Ansprüche 2 bis 5 oder einer Kombination gemäß einem der Ansprüche 6 bis 10, und
- gegebenenfalls Isolieren des Reaktionsprodukts.

14. Verfahren nach Anspruch 13 zur Herstellung eines Aryl- oder Heteroarylamins, wobei eine Verbindung der Formel **(14)**
Ar-X **(14)**
wobei
Ar ein Aryl oder Heteroaryl ist, und
X ein Halogen, ein Sulfonat oder ein Pseudohalogen ist,
mit einem primären oder sekundären Amin umgesetzt wird.
